# EUROPÄISCHE PATENTSCHRIFT

(11) **EP 1 353 660 B1**
(45) Veröffentlichungstag und Bekanntmachung des Hinweises auf die Patenterteilung: **11.01.2006**
(21) Anmeldenummer: 01998336.0
(22) Anmeldetag: 28.11.2001
(51) Int. Cl.: A61K 31/135, A61P 13/10

(54) **VERWENDUNG VON SUBSTITUIERTEN 6-DIMETHYLAMINOMETHYL-1-PHENYL-CYCLOHEXANVERBINDUNGEN ZUR THERAPIE DER HARNINKONTINENZ**
USE OF SUBSTITUTED 6-DIMETHYLAMINOMETHYL-1-PHENYL-CYCLOHEXANE COMPOUNDS FOR TREATING URINARY INCONTINENCE
UTILISATION DE COMPOSES 6-DIMETHYLAMINOMETHYLE-1-PHENYL-CYCLOHEXANE SUBSTITUES POUR TRAITER L'INCONTINENCE URINAIRE

(30) Priorität: 30.11.2000 DE 10059413
(43) Veröffentlichungstag der Anmeldung: 22.10.2003
(73) Patentinhaber: Grünenthal GmbH, 52078 Aachen (DE)
(72) Erfinder: CHRISTOPH, Thomas, 52080 Aachen (DE); FRIDERICHS, Elmar, 52223 Stolberg (DE)
(86) Internationale Anmeldenummer: PCT/EP2001/013917
(87) Internationale Veröffentlichungsnummer: WO 2002/043714

(56) Entgegenhaltungen:
- EP-A- 1 005 861
- DE-A- 10 004 926
- US-A- 4 371 720
- US-A- 5 733 936
- US-A- 5 811 582

## Beschreibung

Die Erfindung betrifft die Verwendung von substituierten 6-Dimethylaminomethyl-1-phenyl-cyclohexanverbindungen als freie Basen und/oder in Form physiologisch verträglicher Salze zur Herstellung eines Arzneimittels zur Behandlung von vermehrtem Harndrang bzw. Harninkontinenz.

Harninkontinenz ist der unwillkürliche Harnabgang. Dieser tritt unkontrolliert auf, wenn der Druck innerhalb der Harnblase den Druck übersteigt, der zum Schließen des Harnleiters notwendig ist. Ursachen können zum einen ein erhöhter interner Blasendruck (z. B. durch Detrusorinstabilität) mit der Folge der Dranginkontinenz und zum anderen ein erniedrigter Sphinkterdruck (z. B. nach Geburt oder chirurgischen Eingriffen) mit der Folge der Streßinkontinenz sein. Der Detrusor ist die grob gebündelte mehrschichtige Blasenwandmuskulatur, deren Kontraktion zur Harnentleerung führt, der Sphinkter der Schließmuskel der Harnröhre. Es treten Mischformen dieser Inkontinenzarten sowie die sogenannte Überflußinkontinenz (z. B. bei benigner Prostatahyperplasie) oder Reflexinkontinenz (z. B. nach Rückenmarksschädigungen) auf. Näheres dazu findet sich bei Chutka, D. S. und Takahashi, P. Y., 1998, Drugs 560: 587-595.

Harndrang ist der auf Harnentleerung (Miktion) abzielende Zustand vermehrter Blasenmuskelspannung bei Annäherung an die Blasenkapazität (bzw. bei deren Überschreitung). Dabei wirkt diese Anspannung als Miktionsreiz. Unter einem vermehrten Harndrang versteht man dabei insbesondere das Auftreten vorzeitigen oder gehäuften manchmal sogar schmerzhaften Harndrangs bis hin zum sog. Harnzwang. Das führt in der Folge zu einer deutlich häufigeren Miktion. Ursachen können u.a. Harnblasenentzündungen und neurogene Blasenstörungen sowie auch Blasentuberkulose sein. Es sind aber noch nicht alle Ursachen geklärt.

Vermehrter Harndrang wie auch Harninkontinenz werden als extrem unangenehm empfunden und es besteht ein deutlicher Bedarf bei von diesen Indikationen betroffenen Personen, eine möglichst langfristige Verbesserung zu erreichen.

Üblicherweise werden vermehrter Harndrang und insbesondere Harninkontinenz medikamentös mit Substanzen behandelt, die an den Reflexen des unteren Harntraktes beteiligt sind (Wein, A. J., 1998, Urology 51 (Suppl. 21): 43 - 47). Meistens sind dies Medikamente, die eine hemmende Wirkung auf den Detrusormuskel, der für den inneren Blasendruck verantwortlich ist, haben. Diese Medikamente sind z. B. Parasympatholytika wie Oxybutynin, Propiverin oder Tolterodin, trizyklische Antidepressiva wie Imipramin oder Muskelrelaxantien wie Flavoxat. Andere Medikamente, die insbesondere den Widerstand der Harnröhre oder des Blasenhalses erhöhen, zeigen Affinitäten zu α-Adrenorezeptoren wie Ephedrin, zu β-Adrenorezeptoren wie Clenbutarol oder sind Hormone wie Östradiol. Auch bestimmte Opioide, Diarylmethylpiperazine und -piperidine-sind für diese Indikation in der WO 93/15062 beschrieben.

Bei den hier in Frage kommenden Indikationen ist zu beachten, daß es sich im allgemeinen um sehr langfristige medikamentöse Anwendungen handelt und sich die Betroffenen im Gegensatz zu vielen Situationen, in denen Analgetika eingesetzt werden, einer sehr unangenehmen, aber nicht unaushaltbaren Situation gegenüber sehen. Daher ist hier - noch mehr als bei Analgetika - darauf zu achten, Nebenwirkungen zu vermeiden, will der Betroffene nicht ein Übel gegen das andere tauschen. Auch sind bei einer dauerhaften Harninkontinenzbehandlung auch analgetische Wirkungen weitgehend unerwünscht.

Aufgabe der vorliegenden Erfindung war es daher, Stoffe aufzufinden, die zur Behandlung von vermehrtem Harndrang bzw. Harninkontinenz hilfreich sind und bei den wirksamen Dosen bevorzugt gleichzeitig geringere Nebenwirkungen und/oder analgetische Wirkungen zeigen als aus dem Stand der Technik bekannt.

Überraschenderweise wurde nun gefunden, daß Verbindungen gemäß allgemeiner Formel I eine hervorragende Wirkung auf die Blasenfunktion besitzen und demzufolge gut zur Behandlung entsprechender Erkrankungen geeignet sind.

Dementsprechend ist Erfindungsgegenstand die Verwendung einer substituierten 6-Dimethylaminomethyl-1-phenyl-cyclohexanverbindung gemäß allgemeiner Formel I, 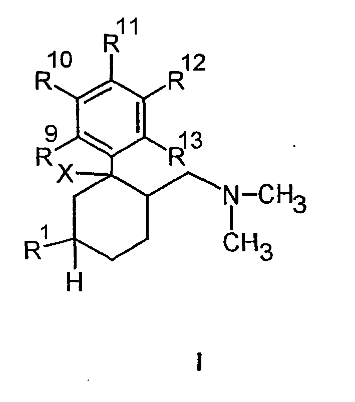
worin
X ausgewählt ist aus OH, F, Cl, H oder OC(O)R⁷ mit R⁷ ausgewählt aus C₁₋₃-Alkyl, verzweigt oder unverzweigt, gesättigt oder ungesättigt, unsubstituiert oder ein- oder mehrfach substituiert,
R¹ ausgewählt ist aus C₁₋₄-Alkyl, Benzyl, CF₃, OH, OCH₂₋C₆H₅, O-C₁₋₄-Alkyl, Cl oder F und
R⁹ bis R¹³ jeweils unabhängig voneinander ausgewählt sind aus H, F, Cl, Br, I, CH₂F, CHF₂, CF₃, OH, SH, OR¹⁴, OCF₃, SR¹⁴, NR¹⁷R¹⁸, SOCH₃, SOCF₃; SO₂CH₃, SO₂CF₃, CN, COOR¹⁴, NO₂, CONR¹⁷R¹⁸; C₁₋₆-Alkyl, verzweigt oder unverzweigt, gesättigt oder ungesättigt, unsubstituiert oder ein- oder mehrfach substituiert; Phenyl, unsubstituiert oder ein- oder mehrfach substituiert;
mit R¹⁴ ausgewählt aus C₁₋₆-Alkyl; Pyridyl, Thienyl, Thiazolyl, Phenyl, Benzyl oder Phenethyl, jeweils unsubstituiert oder ein- oder mehrfach substituiert; PO(O-C₁₋₄-Alkyl)₂, CO(OC₁₋₅-Alkyl), CONH-C₆H₄-(C₁₋₃-Alkyl), CO(C₁₋₅-Alkyl), CO-CHR¹⁷⁻NHR¹⁸, CO-C₆H₄-R¹⁵, mit R¹⁵ ortho-OCOC₁₋₃-Alkyl oder meta- oder para-CH₂N(R¹⁶)₂ mit R¹⁶ C₁₋₄-Alkyl oder 4-Morpholino, wobei in den Resten R¹⁴, R¹⁵ und R¹⁶ die Alkylgruppen verzweigt oder unverzweigt, gesättigt oder ungesättigt, unsubstituiert oder ein- oder mehrfach substituiert sein können;
mit R¹⁷ und R¹⁸ jeweils unabhängig voneinander ausgewählt aus H; C₁₋₆-Alkyl, verzweigt oder unverzweigt, gesättigt oder ungesättigt, unsubstituiert oder ein- oder mehrfach substituiert; Phenyl, Benzyl oder Phenethyl, jeweils unsubstituiert oder ein- oder mehrfach substituiert,
oder
R⁹ und R¹⁰ oder R¹⁰ und R¹¹ zusammen einen OCH₂O-, OCH₂CH₂O-, OCH=CH-, CH=CHO-, CH=C(CH3)O-, OC(CH3)=CH-, (CH₂)₄- oder OCH=CHO-Ring bilden,
in Form ihrer Razemate; Enantiomere, Diastereomere, insbesondere Mischungen ihrer Enantiomere oder Diastereomere oder eines einzelnen Enantiomers oder Diastereomers; ihrer Basen und/oder Salze physiologisch verträglicher Säuren
zur Herstellung eines Arzneimittels zur Behandlung von vermehrtem Harndrang bzw. Harninkontinenz.

Überraschenderweise hatte sich herausgestellt, daß die genannten Substanzen bestimmte physiologische Parameter, die bei vermehrtem Harndrang bzw. Harninkontinenz von Bedeutung sind, deutlich positiv beeinflußen, so entweder den Schwellendruck, das Interkontraktionsintervall, bzw. die Verringerung der rhythmischen Blasenkontraktionen und/oder die Blasenkapazität. Jede einzelne dieser Veränderungen kann eine deutliche Erleichterung im symptomatischen Bild von betroffener Patienten bedeuten. Entsprechende Verbindungen und deren Herstellung sind aus der DE 195 25 137 A1 bekannt.

Im Sinne dieser Erfindung versteht man unter Alkyl-Resten gesättigte und ungesättigte, verzweigte und unverzweigte Kohlenwasserstoffe, die auch mindestens einfach substituiert sein können. Bevorzugte Alkyl-Reste sind Methyl, Ethyl, Vinyl (Ethenyl), Propyl, Allyl (2-Propenyl), 1-Propinyl, Methylethyl, n-Butyl, sek.-Butyl, tert.-Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl, CHF₂, CF₃ oder CH₂OH.

Weiter versteht man unter Cykloalkyl-Resten im Sinne dieser Erfindung gesättigte cyklische Kohlenwasserstoffe, die auch mindestens einfach substituiert sein können. Bevorzugte Cykloalkyl-Reste sind Cyclopropyl, Cyclobutyl, Cyclopentyl, Cyclohexyl oder Cycloheptyl

Dabei versteht man im Zusammenhang mit Alkyl und Cycloalkyl unter dem Begriff substituiert im Sinne dieser Erfindung die Substitution eines Wasserstoffrestes durch F, Cl, Br, I, NH₂, SH oder OH, wobei unter "mehrfach substituiert" zu verstehen ist, daß die Substitution sowohl an verschiedenen als auch an gleichen Atomen mehrfach mit den gleichen oder verschiedenen Substituenten erfolgt, beispielsweise dreifach am gleichen C-Atom wie im Falle von CF₃ oder an verschiedenen Stellen wie im Falle von -CH(OH)-CH=CH-CHCl₂.

Dabei versteht man im Zusammenhang mit Phenyl, Benzyl oder Phenethyl unter substituiert bevorzugt die Substitution mit H, F, Cl, Br, I, CH₂F, CHF₂, CF₃, OH, SH, OR¹⁹, OCF₃, SR¹⁹, NH₂, CONH₂, SOCH₃, SOCF₃, SO₂CH₃, SO₂CF₃, CN, COOR¹⁹, NO₂; C₁₋₆-Alkyl, verzweigt oder unverzweigt, gesättigt oder ungesättigt, unsubstituiert oder ein- oder mehrfach substituiert; Phenyl, unsubstituiert;
mit R19 ausgewählt aus C₁₋₆-Alkyl; verzweigt oder unverzweigt, gesättigt oder ungesättigt, unsubstituiert oder ein- oder mehrfach substituiert; oder C₃₋₇-Cycloalkyl.

Geeignete Salze im Sinne dieser Erfindung und in jeder der beanspruchten Verwendungen sind Salze des jeweiligen Wirkstoffes mit anorganischen bzw. organischen Säuren und/oder einem Zuckeraustauschstoff wie Saccharin, Cyclamat oder Acesulfam. Besonders bevorzugt ist jedoch das Hydrochlorid.

Bevorzugt ist dabei die Verwendung von Verbindungen gemäß Formel I worin X ausgewählt ist aus
OH, F, Cl, OC(O)CH₃ oder H, vorzugsweise OH, F oder H, insbesondere OH.

Weiter bevorzugt ist dabei auch die Verwendung von Verbindungen gemäß Formel I worin R¹ ausgewählt ist aus
C₁₋₄-Alkyl, CF₃, OH, O-C₁₋₄-Alkyl, Cl oder F, vorzugsweise OH, CF₃ oder CH₃,

Bevorzugt ist weiter die Verwendung von Verbindungen gemäß Formel I worin R⁹ bis R¹³, wobei 3 oder 4 der Reste R⁹ bis R¹³ H entsprechen müssen, unabhängig voneinander ausgewählt sind aus
H, Cl, F, OH, CF₂H, CF₃ oder C₁₋₄-Alkyl, gesättigt und unsubstituiert, verzweigt oder unverzweigt; OR¹⁴ oder SR¹⁴, mit R¹⁴ ausgewählt aus C₁₋₃-Alkyl, gesättigt und unsubstituiert, verzweigt oder unverzweigt;
vorzugsweise H, Cl, F, OH, CF₂H, CF₃, OCH₃ oder SCH₃
oder R¹² und R¹¹ einen 3,4-OCH=CH-Ring bilden
insbesondere solche, worin,
wenn R⁹, R¹¹ und R¹³ H entsprechen, einer von R¹⁰ oder R¹² auch H entspricht, während der andere ausgewählt ist aus:
Cl, F, OH, CF₂H, CF₃, OR¹⁴ oder SR¹⁴, vorzugsweise OH, CF₂H, OCH₃ oder SCH₃,
oder,
wenn R⁹ und R¹³ H entsprechen und R¹¹ OH, OCH₃, Cl oder F, vorzugsweise Cl, entspricht, einer von R¹⁰ oder R¹² auch H entspricht, während der andere OH, OCH₃, Cl oder F, vorzugsweise Cl, entspricht,
oder,
wenn R⁹, R¹⁰, R¹² und R¹³ H entsprechen, R¹¹ ausgewählt ist aus CF₃, CF₂H, Cl oder F, vorzugsweise F,
oder,
wenn R¹⁰, R¹¹ und R¹² H entsprechen, einer von R⁹ oder R¹³ auch H entspricht, während der andere ausgewählt ist aus OH, OC₂H₅ oder OC₃H₇.

Ebenfalls bevorzugt ist die Verwendung von Verbindungen gemäß Formel I worin R⁹, R¹¹ und R¹³ H entsprechen, einer von R¹⁰ oder R¹² auch H entspricht, während der andere ausgewählt ist aus:
Cl, F, OH, SH, CF₂H, CF₃, OR¹⁴ oder SR¹⁴, vorzugsweise OH oder OR¹⁴, insbesondere OH oder OC₁₋₃-Alkyl, vorzugsweise OH oder OCH₃.

Es ist auch bevorzugt, wenn Verbindungen gemäß Formel I in Form der Diastereomeren mit der relativen Konfiguration la 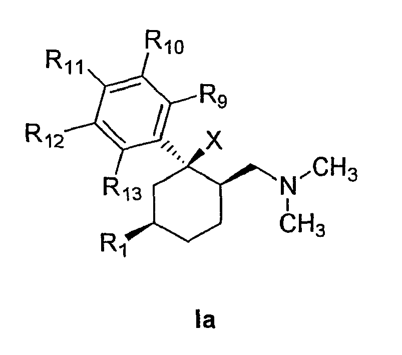
vorliegen, insbesondere in Mischungen mit höherem Anteil dieses Diastereomeren im Vergleich zum anderen Diastereomeren oder als reines Diastereomer verwendet werden.

Weiter ist es bevorzugt, wenn die Verbindungen der Formel I in Form des (+)-Enantiomeren, insbesondere in Mischungen mit höherem Anteil des (+)-Enantiomeren im Vergleich zum (-)-Enantiomeren einer racemischen Verbindung oder als reines (+)-Enantiomer verwendet werden.

Generell ist auch bei bevorzugter Verwendung des (+)-Enantiomeren ein gegenüber dem (+)-Enantiomeren geringerer Anteil an (-)-Enantiomer akzeptabel und darf - muß aber nicht - bei der erfindungsgemässen Verwendung enthalten sein.

Besonders bevorzugt ist die Verwendung einer Verbindung ausgewählt aus folgender Gruppe:
■ (1RS,3RS,6RS)-6-Dimethylaminomethyl-1-(3-methoxyphenyl)-cyclohexan-1,3-diol,
■ (+)-(1R,3R,6R)-6-Dimethylaminomethyl-1-(3-methoxyphenyl)-cyclohexan-1,3-diol,
■ (1RS,3RS,6RS)-6-Dimethylaminomethyl-1-(3-hydroxyphenyl)-cyclohexan-1,3-diol,
■ (1RS,3SR,6RS)-6-Dimethylaminomethyl-1-(3-methoxyphenyl)-cyclohexan-1,3-diol,
■ (+)-(1R,2R,5S)-3-(2-Dimethy)aminomethyl-1-hydroxy-5-methyl-cyclohexyl)-phenol, oder
■ (1RS,2RS,5RS)-3-(2-Dimethylaminomethyl-1-hydroxy-5-trifluoromethyl-cyclohexyl)-phenol,

vorzugsweise als Hydrochlorid.

Auch wenn die erfindungsgemässen Verwendungen lediglich geringe Nebenwirkungen zeigen, kann es beispielsweise zur Vermeidung von bestimmten Formen der Abhängigkeit auch von Vorteil sein, neben Verbindungen gemäß allgemeiner Formel I auch Morphinantagonisten, insbesondere Naloxon, Naltrexon und/oder Levallorphan, zu verwenden.

Geeignete Zusatz- und/oder Hilfsstoffe im Sinne dieser Erfindung sind alle dem Fachmann aus dem Stand der Technik bekannten Stoffe zur Erreichung galenischer Formulierungen. Die Auswahl dieser Hilfsstoffe sowie die einzusetzenden Mengen derselben hängen davon ab, ob das Arzneimittel oral, intravenös, intraperitoneal, intradermal, intramuskulär, intranasal, buccal oder lokal appliziert werden soll. Für die orale Applikation eignen sich Zubereitungen in Form von Tabletten, Kautabletten, Dragees, Kapseln, Granulaten, Tropfen, Säften oder Sirupen, für die parenterale, topische und inhalative Applikation Lösungen, Suspensionen, leicht rekonstituierbare Trockenzubereitungen sowie Sprays. Eine weitere Möglichkeit sind Suppositorien für die Anwendung im Rektum. Die Anwendung in einem Depot in gelöster Form, einer Trägerfolie oder einem Pflaster, gegebenenfalls unter Zusatz von die Hautpenetration fördernden Mitteln, sind Beispiele für geeignete perkutane Applikationsformen. Beispiele für Hilfs- und Zusatzmitteln für die oralen Applikationsformen sind Sprengmittel, Gleitmittel, Binder, Füllmittel, Formtrennmittel, gegebenenfalls Lösungsmittel, Geschmacksstoffe, Zucker, insbesondere Trägermittel, Verdünnungsmittel, Farbstoffe, Antioxidantien etc. Für Suppositorien können u.a. Wachse bzw. Fettsäureester und für parenterale Applikationsmittel Trägerstoffe, Konservierungsmittel, Suspensionshilfsmittel etc. verwendet werden. Die an Patienten zu verabreichenden Wirkstoffmengen variieren in Abhängigkeit vom Gewicht des Patienten, von der Applikationsart und dem Schweregrad der Erkrankung. Aus oral, rektal oder perkutan anwendbaren Zubereitungsformen können die erfindungsgemäßen Verbindungen verzögert freigesetzt werden. Bei der erfindungsgemäßen Indikation sind entsprechende Retard-Formulierungen, insbesondere in Form eines "Once-daily"-Präparats, das nur einmal am Tag eingenommen werden muß, besonders bevorzugt.

Weiter bevorzugt sind Arzneimittel, die wenigstens 0,05 bis 90,0 % des Wirkstoffes enthalten, insbesondere niedrige wirksame Dosierungen, um Neben- oder analgetische Wirkungen zu vermeiden. Üblicherweise werden 0,1 bis 5000 mg/kg, insbesondere 1 bis 500 mg/kg, vorzugsweise 2 bis 250 mg/kg Körpergewicht wenigstens einer Verbindung der Formel I appliziert. Ebenso bevorzugt und üblich ist aber auch die Applikation von 0,01 - 5 mg/kg, vorzugsweise 0,03 bis 2 mg/kg, insbesondere 0,05 bis 1 mg/kg Körpergewicht.

Hilfsstoffe können beispielsweise sein: Wasser, Ethanol, 2-Propanol, Glycerin, Ethylenglycol, Propylenglycol, Polyethylenglycol, Polypropylenglycol, Glucose, Fructose, Lactose, Saccharose, Dextrose, Melasse, Stärke, modifizierte Stärke, Gelatine, Sorbitol, Inositol, Mannitol, mikrokristalline Cellulose, Methylcellulose, Carboxymethylcellulose, Celluloseacetat, Schellack, Cetylalkohol, Polyvinylpyrrolidon, Paraffine, Wachse, natürliche und synthetische Gummis, Akaziengummi, Alginate, Dextran, gesättigte und ungesättigte Fettsäuren, Stearinsäure, Magnesiumstearat, Zinkstearat, Glycerylstearat, Natriumlaurylsulfat, genießbare Öle, Sesamöl, Kokusnußöl, Erdnußöl, Sojabohnenöl, Lecithin, Natriumlactat, Polyoxyethylen- und -propylen-fettsäureester, Sorbitanfettsäureester, Sorbinsäure, Benzoesäure, Citronensäure, Ascorbinsäure, Tanninsäure, Natriumchlorid, Kaliumchlorid, Magnesiumchlorid, Calciumchlorid, Magnesiumoxid, Zinkoxid, Siliciumdioxid, Titanoxid, Titandioxid, Magnesiumsulfat, Zinksulfat, Calciumsulfat, Pottasche, Calciumphosphat, Dicalciumphosphat, Kaliumbromid, Kaliumiodid, Talkum, Kaolin, Pectin, Crospovidon, Agar und Bentonit.

Die Herstellung der erfindungsgemäßen Arzneimittel und pharmazeutischen Zusammensetzungen erfolgt mit Hilfe von im Stand der Technik der pharmazeutischen Formulierung wohlbekannten Mitteln, Vorrichtungen, Methoden und Verfahren, wie sie beispielsweise in "Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind.

So kann z.B. für eine feste Formulierung, wie eine Tablette, der Wirkstoff des Arzneimittels, d.h. eine Verbindung der allgemeinen Struktur I oder eines ihrer pharmazeutisch annehmbaren Salze, mit einem pharmazeutischen Träger, z.B. herkömmlichen Tabletteninhaltsstoffen, wie Maisstärke, Lactose, Saccharose, Sorbitol, Talkum, Magnesiumstearat, Dicalciumphosphat oder pharmazeutisch akzeptable Gummis, und pharmazeutischen Verdünnungsmitteln, wie z. B. Wasser, granuliert werden, um eine feste Zusammensetzungzu bilden, die eine erfindungsgemäße Verbindung oder ein pharmazeutisch annehmbares Salz davon in homogener Verteilung enthält. Unter einer homogenen Verteilung wird hier verstanden, daß der Wirkstoff gleichmäßig über die gesamte Zusammensetzung verteilt ist, so daß diese ohne weiteres in gleich wirksame Einheitsdosis-Formen, wie Tabletten, Pillen oder Kapseln, unterteilt werden kann. Die feste Zusammensetzungwird anschließend in Einheitsdosis-Formen unterteilt. Die Tabletten oder Pillen des erfindungsgemäßen Arzneimittels bzw. der erfindungsgemäßen Zusammensetzungen können auch überzogen oder auf andere Weise kompoundiert werden, um eine Dosisform mit verzögerter Freisetzung bereitzustellen. Geeignete Beschichtungsmittel sind u.a. polymere Säuren und Mischungen von polymeren Säuren mit Materialien wie z.B. Schellack, Cetylalkohol und/oder Celluloseacetat.

Auch wenn die erfindungsgemässen Arzneimittel lediglich geringe Nebenwirkungen zeigen, kann es beispielsweise zur Vermeidung von bestimmten Formen der Abhängigkeit von Vorteil sein, neben den Verbindungen gemäß allgemeiner Formel I auch Morphinantagonisten, insbesondere Naloxon, Naltrexon und/oder Levallorphan, zu verwenden.

Die folgenden Beispiele sollen die Erfindung erläutern.

### Beispiele

### Beispiel 1: Liste der getesteten Substanzen:

Es folgt eine Liste der auf ihre Wirksamkeit getesteten Verbindungen:

| **Name** | **Verbdg**. **Nr**. |
|---|---|
| (1RS,3RS,6RS)-6-Dimethylaminomethyl-1-(3-methoxy-phenyl)-cyclohexan-1,3-diol, Hydrochlorid, | **24** |
| (+)-(1R,3R,6R)-6-Dimethylaminomethyl-1 -(3-methoxy-phenyl)-cyclohexan-1,3-diol, Hydrochlorid, | **25** |
| (1RS,3RS,6RS)-6-Dimethylaminomethyl-1-(3-hydroxy-phenyl)-cyclohexan-1,3-diol, Hydrochlorid, | **26** |
| (1RS,3SR,6RS)-6-Dimethylaminomethyl-1-(3-methoxy-phenyl)-cyclohexan-1,3-diol, Hydrochlorid, | **27** |
| (+)-(1R,2R,55)-3-(2-Dimethylaminomethyl-1-hydroxy-5-methylcyclohexyl)-phenol, Hydrochlorid, | **28** |
| (1RS,2RS,SRS)-3-(2-Dimethylaminomethyl-1-hydroxy-5-trifluoromethylcyclohexyl)-phenol, Hydrochlorid. | **29** |

### Beispiel 2: Testsystem Cystometrie an der wachen naiven Ratte

Es wurden cystometrische Untersuchungen an naiven, weiblichen Sprague-Dawley-Ratten nach der Methode von Ishizuka et. al. ((1997), Naunyn-Schmiedeberg's Arch. Pharmacol. 355: 787 - 793) durchgeführt. Drei Tage nach Implantation von Blasen- und venösen Kathetern wurden die Tiere im wachen Zustand, frei beweglich untersucht. Der Blasenkatheter wurde an einem Druckaufnehmer und eine Injektionspumpe angeschlossen. Die Tiere wurden in Stoffwechselkäfige gesetzt, die die Messung des Harnvolumens ermöglichten. Physiologische Kochsalzlösung wurde in die entleerte Blase infundiert (10 ml/Std.) und Blasendruck und Miktionsvolumen kontinuierlich aufgezeichnet. Nach einer Stabilisierungsphase wurde eine 20minütige Phase aufgezeichnet, die durch normale, reproduzierbare Miktionszyklen gekennzeichnet war. Es wurden unter anderem die folgenden Parameter bestimmt:
■ Schwellendruck (threshold pressure TP, Blasendruck unmittelbar vor Miktion),
■ Blasenkapazität (bladder capacity BC, Restvolumen nach vorhergehender Miktion plus Volumen der infudierten Lösung während der Füllungsphase),
■ Interkontraktionsintervall (inter-contraction interval (ICI), das Zeitintervall zwischen den Miktionen).

Eine Erhöhung des Schwellendrucks (TP) zeigt eine wichtige therapeutische Wirkung bei einer der erfindungsgemässen Indikationen an. Auch das Interkontraktionsintervall (ICI) ist ein wichtiger Parameter zur Messung der physiologischen Wirksamkeit eines Stoffes in der Behandlung der Harninkontinenz, ebenso wie die Blasenkapazität (BC). Dabei ist es für eine Wirksamkeit aufgrund der sehr heterogenen Ursachen für die Symptomatik dieser Erkrankungsbilder nicht nötig, alle drei Parameter positiv zu beeinflussen. Es genügt daher völlig, wenn nur in einem dieser Parameter eine posive Wirkung festzustellen ist, um in der Harninkontinenz oder vermehrtem Harndrang einsetzbar zu sein.

Nach der Aufzeichnung von drei reproduzierbaren Miktionszyklen als Vorwert, wurden die Testsubstanzen **24** (1,0; 3,0; 5,0 mg/kg), **25** (1,5 mg/kg) und **26** (3,0 mg/kg) im Vehikel = 0.9 % NaCl i.v. appliziert und die Wirkung auf die cystometrischen Parameter 90 bis 120 Minuten aufgezeichnet. Im Wirkmaximum wurde der Mittelwert von 3 Miktionszyklen bestimmt und als prozentuale Veränderung gegenüber dem Vorwert dargestellt (Tabelle 1).

**Tabelle 1: Beeinflussung der cystometrischen Parameter durch die Testsubstanzen (Veränderung zum Vorwert [%]); n entspricht der Anzahl der Versuchstiere.**

| **Verbindung:** (Konzentration) | **TP** threshold pressure | **BC** bladder capacity | **ICI** inter-contraction interval |
|---|---|---|---|
| **24** | | | |
| 1,0 mg/kg iv (n=7) | +44,0 %*** | -8,0 % | -15 %** |
| 3,0 mg/kg iv (n=8) | +94,0 %** | -16,0 %* | -16 %* |
| 5,0 mg/kg iv (n=8) | +69,0 %* | -26,0 %* | -21,2 % |
| **25** | | | |
| 1,5 mg/kg iv (n=8) | +62,0 %* | -14,0 %* | -9,0 % |
| **26** | | | |
| 3,0 mg/kg iv (n=7) | +86,0 %*** | +29,0 %* | +27,0 %* |

Die untersuchten Substanzen zeigen eine positive Wirkung auf die Blasenregulation und sind somit geeignet zur Behandlung der Harninkontinenz.

### Beispiel 3. Testsystem Cystometrie an der narkotisierten naiven Ratte

Die cystometrische Untersuchung an naiven weiblichen Ratten wurde nach der Methode von Kimura et al. (Kimura et al., 1996, Int. J. Urol. 3:218-227) durchgeführt. An narkotisierten, ventilierten Ratten wird das Abdomen eröffnet und die Harnleiter abgebunden. Der Harn wird von den Nieren abgeleitet. Ein Katheter wird in die Blase eingeführt und fixiert. Über diesen wird Saline mittels Infusionspumpe in die Blase infundiert, bis diese rhythmische Spontanaktivität in Form von Kontraktionen zeigt, welche über einen angeschlossenen Druckaufnehmer aufgenommen werden können. Die Testsubstanz wird nach Erreichen stabiler Ausgangswerte in kumulativer Weise i.v. appliziert. Eine Beeinflussung der Blasenfunktion äußert sich über die Unterdrückung der Spontankontraktionen. Dabei gilt als Parameter für die Unterdrückung das Ausbleiben der Kontraktionen über einen Zeitraum von 10 min.

Bei allen hier aufgelisteten Substanzen war eine Unterdrückung der Spontankontraktionen in den Ratten meßbar, wobei Tabelle 2 den Mittelwert der niedrigsten Dosis aus mindestens 2 Versuchen angibt, bei der erstmals Kontraktionen über einen Zeitraum von 10 min ausbleiben.

**Tabelle 2: (n entspricht der Anzahl der in den Wert eingegangenen Versuche)**

| **Verbdg.-Nr.** | **Niedrigste Dosis (mg/kg)** |
|---|---|
| **27** | 115 (n=2) |
| **28** | 16,7 (n=3) |
| **29** | 23,3 (n=3) |

Die untersuchten Substanzen zeigen eine positive Wirkung auf die Blasenregulation und sind somit geeignet zur Behandlung der Harninkontinenz.

### Beispiel 4: Parenterale Applikationsform

1 g der Verbindung **26** wird in 1 l Wasser für Injektionszwecke bei Raumtemperatur gelöst und anschließend durch Zugabe von NaCl auf isotone Bedingungen eingestellt.

## Patentansprüche

1. Verwendung einer substituierten 6-Dimethylaminomethyl-1-phenylcyclohexanverbindung gemäß allgemeiner Formel I, 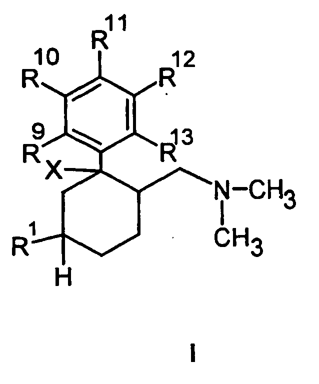
worin
X ausgewählt ist aus OH, F, Cl, H oder OC(O)R⁷ mit R⁷ ausgewählt aus C₁₋₃-Alkyl, verzweigt oder unverzweigt, gesättigt oder ungesättigt, unsubstituiert oder ein- oder mehrfach substituiert,
R¹ ausgewählt ist aus C₁₋₄-Alkyl, Benzyl, CF₃, OH, OCH₂₋C₆H₅, O-C₁₋₄-Alkyl, Cl oder F und
R⁹ bis R¹³ jeweils unabhängig voneinander ausgewählt sind aus H, F, Cl, Br, I, CH₂F, CHF₂, CF₃, OH, SH, OR¹⁴, OCF₃, SR¹⁴, NR¹⁷R¹⁸, SOCH₃, SOCF₃; SO₂CH₃, SO₂CF₃, CN, COOR¹⁴, NO₂, CONR¹⁷R¹⁸; C₁₋₆-Alkyl, verzweigt oder unverzweigt, gesättigt oder ungesättigt, unsubstituiert oder ein- oder mehrfach substituiert; Phenyl, unsubstituiert oder ein- oder mehrfach substituiert;
mit R¹⁴ ausgewählt aus C₁₋₆-Alkyl; Pyridyl, Thienyl, Thiazolyl, Phenyl, Benzyl oder Phenethyl, jeweils unsubstituiert oder ein- oder mehrfach substituiert; PO(O-C₁₋₄-Alkyl)₂, CO(OC₁₋₅-Alkyl), CONH-C₆H₄-(C₁₋₃-Alkyl), CO(C₁₋₅-Alkyl), CO-CHR¹⁷⁻NHR¹⁸, CO-C₆H₄-R¹⁵, mit R¹⁵ ortho-OCOC₁₋₃-Alkyl oder meta- oder para-CH₂N(R¹⁶)₂ mit R¹⁶ C₁₋₄-Alkyl oder 4-Morpholino, wobei in den Resten R¹⁴, R¹⁵ und R¹⁶ die Alkylgruppen verzweigt oder unverzweigt, gesättigt oder ungesättigt, unsubstituiert oder ein- oder mehrfach substituiert sein können;
mit R¹⁷ und R18 jeweils unabhängig voneinander ausgewählt aus H; C₁₋₆-Alkyl, verzweigt oder unverzweigt, gesättigt oder ungesättigt, unsubstituiert oder ein- oder mehrfach substituiert; Phenyl, Benzyl oder Phenethyl, jeweils unsubstituiert oder ein- oder mehrfach substituiert,
oder
R⁹ und R¹⁰ oder R¹⁰ und R¹¹ zusammen einen OCH₂O-, OCH₂CH₂O-, OCH=CH-, CH=CHO-, CH=C(CH3)O-, OC(CH3)=CH-, (CH₂)₄- oder OCH=CHO-Ring bilden,
in Form ihrer Razemate; Enantiomere, Diastereomere, insbesondere Mischungen ihrer Enantiomere oder Diastereomere oder eines einzelnen Enantiomers oder Diastereomers; ihrer Basen und/oder Salze mit physiologisch verträglichen Säuren
zur Herstellung eines Arzneimittels zur Behandlung von vermehrtem Harndrang bzw. Harninkontinenz.

2. Verwendung gemäß Anspruch 1, **dadurch gekennzeichnet, daß** X ausgewählt ist aus
OH, F, Cl, OC(O)CH₃ oder H, vorzugsweise OH, F oder H, insbesondere OH.

3. Verwendung gemäß einem der Ansprüche 1 oder 2, **dadurch gekennzeichnet, daß** R¹ ausgewählt ist aus
C₁₋₄-Alkyl, CF₃, OH, O-C₁₋₄-Alkyl, Cl oder F, vorzugsweise OH, CF₃ oder CH₃.

4. Verwendung gemäß einem der Ansprüche 1 bis 3, **dadurch gekennzeichnet, daß** R⁹ bis R¹³, wobei 3 oder 4 der Reste R⁹ bis R¹³ H entsprechen müssen, unabhängig voneinander ausgewählt sind aus
H, Cl, F, OH, CF₂H, CF₃ oder C₁₋₄-Alkyl, gesättigt und unsubstituiert, verzweigt oder unverzweigt; OR¹⁴ oder SR¹⁴, mit R¹⁴ ausgewählt aus C₁₋₃-Alkyl, gesättigt und unsubstituiert, verzweigt oder unverzweigt;
vorzugsweise H, Cl, F, OH, CF₂H, CF₃, OCH₃ oder SCH₃ oder R¹² und R¹¹ einen 3,4-OCH=CH-Ring bilden
insbesondere **dadurch gekennzeichnet, daß**,
wenn R⁹, R¹¹ und R¹³ H entsprechen, einer von R¹⁰ oder R¹² auch H entspricht, während der andere ausgewählt ist aus:
Cl, F, OH, CF₂H, CF₃, OR¹⁴ oder SR¹⁴, vorzugsweise OH, CF₂H, OR¹⁴ oder SCH₃, insbesondere OH oder OC₁₋₃₋Alkyl, vorzugsweise OH oder OCH₃,
oder,
wenn R⁹ und R¹³ H entsprechen und R¹¹ OH, OCH₃, Cl oder F, vorzugsweise Cl, entspricht, einer von R¹⁰ oder R¹² auch H entspricht, während der andere OH, OCH₃, Cl oder F, vorzugsweise Cl, entspricht,
oder,
wenn R⁹, R¹⁰, R¹² und R¹³ H entsprechen, R¹¹ ausgewählt ist aus CF₃, CF₂H, Cl oder F, vorzugsweise F,
oder,
wenn R¹⁰, R¹¹ und R¹² H entsprechen, einer von R⁹ oder R¹³ auch H entspricht, während der andere ausgewählt ist aus OH, OC₂H₅ oder OC₃H₇.

5. Verwendung gemäß einem der Ansprüche 1 bis 4, **dadurch gekennzeichnet, daß** R⁹, R¹¹ und R¹³ H entsprechen, einer von R¹⁰ oder R¹² auch H entspricht, während der andere ausgewählt ist aus:
Cl, F, OH, SH, CF₂H, CF₃, OR¹⁴ oder SR¹⁴, vorzugsweise OH oder OR¹⁴, insbesondere OH oder OC₁₋₃-Alkyl, vorzugsweise OH oder OCH₃.

6. Verwendung gemäß einem der Ansprüche 1 bis 5, **dadurch gekennzeichnet, daß** Verbindungen der Formel I in Form der Diastereomeren mit der relativen Konfiguration la 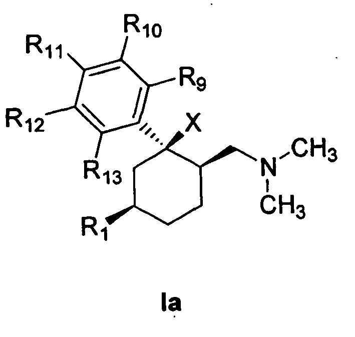
vorliegen, insbesondere in Mischungen mit höherem Anteil dieses Diastereomeren im Vergleich zum anderen Diastereomeren oder als reines Diastereomer verwendet werden.

7. Verwendung gemäß einem der Ansprüche 1 bis 6, **dadurch gekennzeichnet, daß** die Verbindungen der Formel I in Form des (+)-Enantiomeren, insbesondere in Mischungen mit höherem Anteil des (+)-Enantiomeren im Vergleich zum (-)-Enantiomeren einer racemischen Verbindung oder als reines (+)-Enantiomer verwendet werden.

8. Verwendung gemäß einem der Ansprüche 1 bis 7, **dadurch gekennzeichnet, daß** eine Verbindung ausgewählt aus folgender Gruppe verwendet wird:
■ (1RS,3RS,6RS)-6-Dimethylaminomethyl-1-(3-methoxyphenyl)-cyclohexan-1,3-diol,
■ (+)-(1R,3R,6R)-6-Dimethylaminomethyl-1-(3-methoxyphenyl)-cyclohexan-1,3-diol,
■ (1RS,3RS,6RS)-6-Dimethylaminomethyl-1-(3-hydroxyphenyl)-cyclohexan-1,3-diol,
■ (1RS,3SR,6RS)-6-Dimethylaminomethyl-1-(3-methoxyphenyl)-cyclohexan-1,3-diol,
■ (+)-(1R,2R,5S)-3-(2-Dimethylaminomethyl-1-hydroxy-5-methyl-cyclohexyl)-phenol oder
■ (1RS,2RS,5RS)-3-(2-Dimethylaminomethyl-1-hydroxy-5-trifluoromethyl-cyclohexyl)-phenol,
vorzugsweise als Hydrochlorid.

## Claims

1. Use of a substituted 6-dimethylaminomethyl-1-phenylcyclohexane compound according to the general formula I 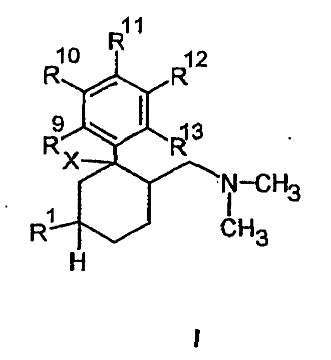
wherein
X is selected from OH, F, Cl, H or OC(O)R⁷ where R⁷ is selected from C₁₋₃-alkyl that is branched or unbranched, saturated or unsaturated, unsubstituted or singly or multiply substituted,
R¹ is selected from C₁₋₄-alkyl, benzyl, CF₃, OH, OCH₂-C₆H₅, O-C₁₋₄-alkyl, Cl or F and
R⁹ to R¹³ are in each case selected independently of one another from H, F, Cl, Br, I, CH₂F, CHF₂, CF₃, OH, SH, OR¹⁴, OCF₃, SR¹⁴, NR¹⁷R¹⁸, SOCH₃, SOCF₃; SO₂CH₃, SO₂CF₃, CN, COOR¹⁴, NO₂, CONR¹⁷R¹⁸; C₁₋₆-alkyl that is branched or unbranched, saturated or unsaturated, unsubstituted or singly or multiply substituted; phenyl that is unsubstituted or singly or multiply substituted;
where R¹⁴ is selected from C₁₋₆-alkyl; pyridyl, thienyl, thiazolyl, phenyl, benzyl or phenethyl, in each case unsubstituted or singly or multiply substituted; PO(O-C₁₋₄₋alkyl)₂, CO(OC₁₋₅-alkyl), CONH-C₆H₄-(C₁₋₃₋alkyl), CO(C₁₋₅-alkyl), CO-CHR¹⁷-NHR¹⁸, CO-C₆H₄-R¹⁵, where R¹⁵ is ortho-OCOC₁₋₃-alkyl or meta- or para-CH₂N(R¹⁶)₂ where R¹⁶ is C₁₋₄₋alkyl or 4-morpholino, wherein in the radicals R¹⁴, R¹⁵ and R¹⁶ the alkyl groups may be branched or unbranched, saturated or unsaturated, unsubstituted or singly or multiply substituted;
where R¹⁷ and R¹⁸ are in each case selected independently of one another from H; C₁₋₆₋alkyl that is branched or unbranched, saturated or unsaturated, unsubstituted or singly or multiply substituted; phenyl, benzyl or phenethyl that is in each case unsubstituted or singly or multiply substituted,
or
R⁹ and R¹⁰ or R¹⁰ and R¹¹ together form an OCH₂O, OCH₂CH₂O, OCH=CH, CH=CHO, CH=C(CH₃)O, OC(CH₃)=CH, (CH₂)₄ or OCH=CHO ring,
in the form of their racemates; enantiomers, diastereomers, in particular mixtures of their enantiomers or diastereomers, or an individual enantiomer or diastereomer; their bases and/or salts with physiologically compatible acids
for the production of a medicament for treating increased urinary urgency or urinary incontinence.

2. Use according to claim 1, **characterised in that** X is selected from
OH, F, Cl, OC (O) CH₃ or H, preferably OH, F or H, in particular OH.

3. Use according to one of claims 1 or 2, **characterised in that** R¹ is selected from
C₁₋₄-alkyl, CF₃, OH, O-C₁₋₄-alkyl, Cl or F, preferably OH, CF₃ or CH₃.

4. Use according to one of claims 1 to 3, **characterised in that** R⁹ to R¹³, wherein three or four of the radicals R⁹ to R¹³ must correspond to H, are selected independently of one another from
H, Cl, F, OH, CF₂H, CF₃ or C₁₋₄-alkyl that is saturated and unsubstituted, branched or unbranched; OR¹⁴ or SR¹⁴ where R¹⁴ is selected from C₁₋₃-alkyl that is saturated and unsubstituted, branched or unbranched;
preferably H, Cl, F, OH, CF₂H, CF₃, OCH₃ or SCH₃
or R¹² and R¹¹ form a 3,4-OCH=CH ring
in particular **characterised in that**
if R⁹, R¹¹ and R¹³ correspond to H, then one of R¹⁰ or R¹² also corresponds to H, while the other is selected from:
Cl, F, OH, CF₂H, CF₃, OR¹⁴ or SR¹⁴, preferably OH, CF₂H, OR¹⁴ or SCH₃, in particular OH or OC₁₋₃-alkyl, preferably OH or OCH₃,
or,
if R⁹ and R¹³ correspond to H and R¹¹ corresponds to OH, OCH₃, Cl or F, preferably Cl, then one of R¹⁰ or R¹² also corresponds to H, while the other corresponds to OH, OCH₃, Cl or F, preferably Cl,
or,
if R⁹, R¹⁰, R¹² and R¹³ correspond to H, R¹¹ is selected from CF₃, CF₂H, Cl or F, preferably F,
or,
if R¹⁰, R¹¹ and R¹² correspond to H, one of R⁹ or R¹³ also corresponds to H, while the other is selected from OH, OC₂H₅ or OC₃H₇.

5. Use according to one of claims 1 to 4, **characterised in that** R⁹, R¹¹ and R¹³ correspond to H, one of R¹⁰ or R¹² also corresponds to H, while the other is selected from:
Cl, F, OH, SH, CF₂H, CF₃, OR¹⁴ or SR¹⁴, preferably OH or OR¹⁴, in particular OH or OC₁₋₃-alkyl, preferably OH or OCH₃.

6. Use according to one of claims 1 to 5, **characterised in that** compounds of the formula I are present in the form of the diastereomers with the relative configuration Ia 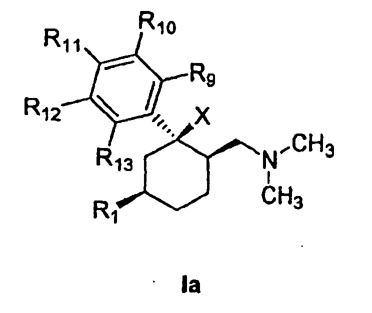
in particular in mixtures with a higher proportion of this diastereomer compared to the other diastereomer or as pure diastereomer.

7. Use according to one of claims 1 to 6, **characterised in that** the compounds of formula I are employed in the form of the (+) enantiomer, in particular in mixtures with a higher proportion of the (+) enantiomer compared to the (-) enantiomer of a racemic compound, or as pure (+) enantiomer.

8. Use according to one of claims 1 to 7, **characterised in that** a compound selected from the following group is used:
• (1RS,3RS,6RS)-6-dimethylaminomethyl-1-(3-methoxyphenyl)-cyclohexane-1,3-diol,
• (+)-(1R, 3R, 6R)-6-dimethylaminomethyl-1-(3-methoxyphenyl)-cyclohexane-1,3-diol,
• (1RS,3RS,6RS)-6-dimethylaminomethyl-1-(3-hydroxyphenyl)-cyclohexane-1,3-diol,
• (1RS,3SR,6RS)-6-dimethylaminomethyl-1-(3-methoxyphenyl)-cyclohexane-1,3-diol,
• (+)-(1R,2R,5S)-3-(2-dimethylaminomethyl-1-hydroxy-5-methylcyclohexyl)-phenol, or
• (1RS,2RS,5RS)-3-(2-dimethylaminomethyl-1-hydroxy-5-trifluoromethylcyclohexyl)-phenol
preferably as hydrochloride.

## Revendications

1. Utilisation d'un composé substitué de 6-diméthylaminométhyl-1-phényl-cyclohexane de formule générale I ; 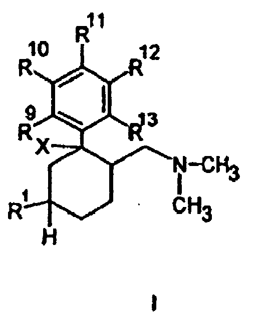
dans laquelle
X est choisi entre OH, F, Cl, H ou un groupe OC(O)R⁷ dans lequel R⁷ est choisi entre des restes alkyle en C₁ à C₃, ramifiés ou non ramifiés, saturés ou non saturés, non substitués ou portant un ou plusieurs substituants,
R¹ est choisi entre un reste alkyle en C₁ à C₄, benzyle, CF₃, OH, OCH₂-C₆H₅, O-alkyle en C₁ à C₄, Cl ou F et
R⁹ à R¹³ sont choisis, chacun indépendamment des autres, entre H, F, Cl, Br, I, CH₂F, CHF₂, CF₃, OH, SH, OR¹⁴, OCF₃, SR¹⁴, NR¹⁷R¹⁸, SOCH₃, SOCF₃ ; SO₂CH₃, SO₂CF₃, CN, COOR¹⁴, NO₂, CONR¹⁷R¹⁸ ; alkyle en C₁ à C₆, ramifié ou non ramifié, saturé ou non saturé, non substitué ou portant un ou plusieurs substituants ; phényle non substitué ou portant un ou plusieurs substituants ;
R¹⁴ étant choisi entre un reste alkyle en C₁ à C₆ ; pyridyle, thiényle, thiazolyle, phényle, benzyle ou phénéthyle, chacun non substitué ou portant un ou plusieurs substituants ; un reste PO(O-alkyle en C₁ à C₄)₂, CO(O-alkyle en C₁ à C₅), CONH-C₆H₄-(alkyle en C₁ à C₃), CO(alkyle en C₁ à C₅), CO-CHR¹⁷-NHR¹⁸, CO-C₆H₄R¹⁵, R¹⁵ étant un reste ortho-OCO(alkyle en C₁ à C₃) ou méta- ou para-CH₂N(R¹⁶)₂ où R¹⁶ est un radical alkyle en C₁ à C₄ ou 4-morpholino, les groupes alkyle présents dans les restes R¹⁴, R¹⁵ et R¹⁶ étant ramifiés ou non ramifiés, saturés ou non saturés, non substitués ou porteurs d'un ou plusieurs substituants ;
R¹⁷ et R¹⁸ étant choisis, indépendamment l'un de l'autre entre H ; un radical alkyle en C₁ à C₆, ramifié ou non ramifié, saturé ou non saturé, non substitué ou substitué une ou plusieurs fois ; phényle, benzyle ou phénéthyle, chacun non substitué ou substitué une ou plusieurs fois,
ou bien
R⁹ et R¹⁰ ou R¹⁰ et R¹¹ forment ensemble un noyau OCH₂O-OCH₂CH₂O-, OCH=CH-, CH=CHO-, CH=C(CH₃)O-, OC(CH₃)=CH-, (CH₂)₄- ou OCH=CHO
sous forme de leurs racémates ; leurs énantiomères, leurs diastéréoisomères, en particulier sous forme de mélanges de leurs énantiomères ou diastéréosiomères ou d'un énantiomère ou diastéréoisomère individuel ; de leurs bases et/ou de leurs sels formés avec des acides acceptables du point de vue physiologique,
pour la préparation d'un médicament destiné au traitement de la répétition de besoins impérieux d'uriner, ou de l'incontinence d'urine.

2. Utilisation suivant la revendication 1, **caractérisée en ce que** X est choisi entre
OH, F, Cl, OC(O)CH₃ ou H, avantageusement OH, F ou H, notamment OH.

3. Utilisation suivant l'une des revendications 1 ou 2, **caractérisée en ce que** R¹ est choisi entre
alkyle en C₁ à C₄, CF₃, OH, O-alkyle en C₁ à C₄, Cl ou F, avantageusement OH, CH₃ ou CH₃.

4. Utilisation suivant l'une des revendications 1 à 3, **caractérisée en ce que** R⁹ à R¹³, dont 3 ou 4 d'entre eux doivent correspondre à H, sont choisis indépendamment les uns des autres entre
H, Cl, F, OH, CF₂H, CF₃ ou alkyle en C₁ à C₄, saturé et non saturé, ramifié ou non ramifié ; OR¹⁴ ou SR¹⁴, R¹⁴ étant choisi entre des radicaux alkyle en C₁ à C₃, saturés et non substitués, ramifiés ou non ramifiés ;
avantageusement H, Cl, F, OH, CF₂H, CF₃, OCH₃ ou SCH₃
ou bien R¹² et R¹¹ forment un noyau 3, 4-OCH=CH-,
**caractérisée en** particulier par le fait que
lorsque R⁹, R¹¹ et R¹³ correspondent à H, l'un de R¹⁰ ou R¹² correspond aussi à H, tandis que l'autre est choisi entre :
Cl, F, OH, CF₂H, CF₃, OR¹⁴ ou SR¹⁴, avantageusement OH, CF₂H, OR¹⁴ ou SCH₃, en particulier OH ou O-alkyle en C₁ à C₃, notamment OH ou OCH₃.
ou bien
lorsque R⁹ et R¹³ correspondent à H et R¹¹ correspond à OH, OCH₃, Cl ou F, avantageusement Cl, l'un de R¹⁰ ou R¹² correspond aussi à H, tandis que l'autre correspond à OH, OCH₃, Cl ou F, avantageusement Cl,
ou bien
lorsque R⁹, R¹⁰, R¹² et R¹³ correspondent à H, R¹¹ est choisi entre CF₃, CF₂H, Cl ou F, et représente avantageusement F,
ou bien
lorsque R¹⁰, R¹¹ et R¹² correspondent à H, l'un de R⁹ ou R¹³ correspond aussi à H tandis que l'autre est choisi entre OH, OC₂H₅ ou OC₃H₇.

5. Utilisation suivant l'une des revendications 1 à 4, **caractérisée en ce que** R⁹, R¹¹ et R¹³ correspondent à H, l'un de R¹⁰ ou R¹² correspond aussi à H, tandis que l'autre est choisi entre : Cl, F, OH, SH, CF₂H, CF₃, OR¹⁴ ou SR¹⁴, avantageusement OH ou OR¹⁴, en particulier OH ou O-alkyle en C₁ à C₃, notamment OH ou OCH₃.

6. Utilisation suivant l'une des revendications 1 à 5, **caractérisée en ce qu'**on utilise des composés de formule I sous forme des diastétéoisomères ayant la configuration relative Ia 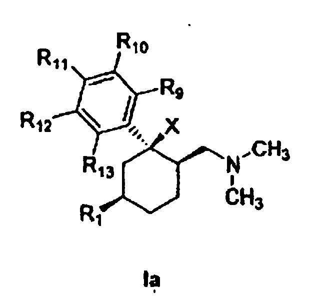
en particulier dans des mélanges contenant une plus forte proportion de ce diastéréoisomère comparativement à l'autre diastéréoisomère, ou sous forme du diastéréoisomère pur.

7. Utilisation suivant l'une des revendications 1 à 6, **caractérisée en ce que** les composés de formule I sont utilisés sous forme du (+)-énantiomère, en particulier dans des mélanges contenant une plus forte proportion du (+)-énantiomère comparativement au (-)-énantiomère d'un composé racémique ou sous forme du (+)-énantiomère pur.

8. Utilisation suivant l'une des revendications 1 à 7, **caractérisée en ce qu'**on utilise un composé choisi dans le groupe des composés suivants :
■ (1RS,3RS,6RS)-6-diméthylaminométhyl-1-(3-méthoxy-phényl)-cyclohexane-1,3-diol,
■ (+)-(1R,3R,6R)-6-diméthylaminométhyl-1-(3-méthoxyphényl)-cyclohexane-1,3-diol,
■ (1RS,3RS,6RS)-6-diméthylaminométhyl-1-(3-hydroxy-phényl)-cyclohexane-1,3-diol,
■ (1RS,3RS,6RS)-6-diméthylaminométhyl-1-(3-méthoxy-phényl)-cyclohexane-1,3-diol,
■ (+)-(1R,2R,5S)-3-(2-diméthylaminométhyl-1-hydroxy-5-méthyl-cyclohexyl)-phénol ou
■ (1RS,2RS,5RS)-3-(2-diméthylaminométhyl-1-hydroxy-5-tri-fluorométhyl-cyclohexyl)-phénol,
avantageusement sous forme du chlorhydrate.
